# EUROPÄISCHE PATENTSCHRIFT

(11) **EP 0 470 050 B1**
(45) Veröffentlichungstag und Bekanntmachung des Hinweises auf die Patenterteilung: **02.11.1995**
(21) Anmeldenummer: 91810603.0
(22) Anmeldetag: 26.07.1991
(51) Int. Cl.: C07D 251/42

(54) **Verfahren zur Herstellung von 2-Amino-4-alkoxy-s-triazinen**
Process for the preparation of 2-amino-4-alkoxy-s-triazines
Procédé pour la préparation d'amino-2 alkoxy-4 triazines-s

(30) Priorität: 03.08.1990 US 562161
(43) Veröffentlichungstag der Anmeldung: 05.02.1992
(73) Patentinhaber: CIBA-GEIGY AG, 4002 Basel (CH)
(72) Erfinder: Oliver, Michael A., Baton Rouge, LA 70808 (US); Oliver, Ward H., Baton Rouge, LA 70810 (US)

(56) Entgegenhaltungen:
- DE-A- 3 411 202
- US-A- 3 154 547
- US-A- 4 587 337
- US-A- 4 886 881
- CHEMICAL ABSTRACTS, Band 59, Nr. 4, 19. August 1963, Columbus, Ohio, USA K. ROBERT HUFFMAN et al.: "N-Cyanoimidates" Spalte 3927, Zusammenfassung-Nr. 3927g
- CHEMICAL ABSTRACTS, Band 78, Nr. 25, 25. Juni 1973, Columbus, Ohio, USAFURUKAWA, MITSURU et al. "Reaction of biguanides and related compounds. VI.Con- densation of N-amidino-O- alkylisoureas with beta-di- -ketones" Seite 413,Spalte 2, Zu- sammenfassung-Nr. 159 566w
- CHEMICAL ABSTRACTS, Band 98, Nr. 23, 6. Juni 1983, Columbus, Ohio, USA PEREZ,MIGUEL A. et al. "Preparation of substituted 5-pyrimidinecarbonitriles and 1,3,5-triazines from alkyl N-cyanoimidates" Seite 650, Spalte 1, Zu- sammenfassung-Nr. 198 166y
- CHEMICAL ABSTRACTS, Band 86, Nr. 13, 28. MUrz 1977, Columbus, Ohio, USAFUCHIGAMI, TOSHIO et al. "N-Halogen compounds of cyanamide derivatives. VI. Thepreparation and reaction of 2-carbonimidoyl-3-imino- - 4-1,2,4-thiadiazolines"Seite 534, Spalte 2, Zu- sammenfassung-Nr. 89 709j
- CHEMICAL ABSTRACTS, Band 110, Nr. 5, 30. JUnner 1989, Columbus, Ohio, USAKAVALEK, JAROMIR et al. "Process for the production of 2-amino-4-alkoxy-6-alkyl-1,3,5-triazines, useful as veterinary sedatives and herbicide intermediates" Seite 649, Spalte 2, Zusammenfassung-Nr. 39 026c.

## Beschreibung

Die vorliegende Erfindung betrifft ein Verfahren zur Herstellung von 2-Amino-4-alkoxy-s-triazinen. Insbesondere betrifft die vorliegende Erfindung ein Verfahren zur Herstellung von 2-Amino-4-alkoxy-s-triazinen, welche durch eine Alkyl-, Aryl- oder Aralkylgruppe in der 6-Position substituiert sind, das dadurch gekennzeichnet ist, daß man ein N-cyanoimido-dialkylcarbonat mit einem Amidinsalz in Gegenwart eines Lösungsmittels und einer starken Base umsetzt.

2-Amino-4-alkoxy-s-triazine sind wichtige Produkte, die als pharmakologische Mittel und als Zwischenprodukte für Herbizide, Farbstoffe und optische Aufheller Verwendung finden. Viele Synthesewege für derartige Verbindungen sind bekannt und werden beispielsweise in den U.S. Patenten Nr. 3,154,547, 4,587,337 und 4,886,881 diskutiert. Im allgemeinen können die Verfahren aus dem Stand der Technik für die Herstellung im industriellen Maßstab aus der Sicht niedriger Gesamtausbeuten ausgehend von leicht verfügbaren Ausgangsmaterialien, verlängerter Reaktionszyklusdauer, Kosten, Sicherheit und/oder Umweltproblemen nicht befriedigen.

Es wurden nun gefunden, daß man 2-Amino-4-alkoxy-s-triazine, welche durch eine gegebenenfalls substituierte Alkyl-, Aryl- oder Aralkylgruppe in der 6-Position substituiert sind, in guter Ausbeute und Reinheit in der Weise herstellen kann, indem man ein N-cyanoimido-dialkylcarbonat mit einem Amidinsalz in geeigneter Weise miteinander umsetzt.

Gegenstand der vorliegenden Erfindung ist somit ein Verfahren zur Herstellung von 2-Amino-4-alkoxy-s-triazinen der Formel I 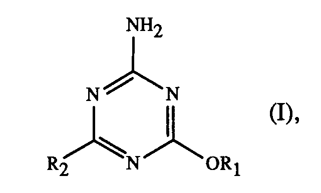
worin
R₁ für C₁-C₄-Alkyl oder C₁-C₂-Alkoxy-C₁-C₂-alkyl; und R₂ für C₁-C₈-Alkyl, welches unsubstituiert ist oder durch C₁-C₄-Alkoxy, Phenyl oder durch C₁-C₄-Alkyl, C₁-C₄-Alkoxy, Difluormethyl, Trifluormethyl, Halogen oder Nitro substituiertes Phenyl substituiert ist; Phenyl oder durch C₁-C₄-Alkyl, C₁-C₄-Alkoxy, Difluormethyl, Trifluormethyl, Halogen oder Nitro substituiertes Phenyl; C₃-C₆-Cycloalkyl oder Allyl stehen, durch Umsetzung eines N-cyanoimido-dialkylcarbonats der Formel II 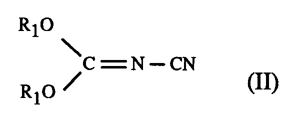
worin R₁ wie in Formel I definiert ist, mit einem
Amidinsalz der Formel III 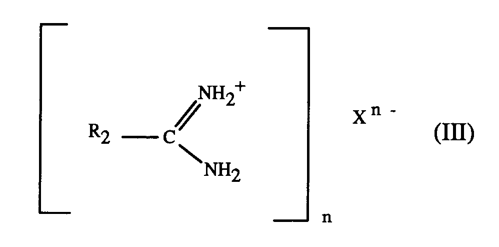
in welcher R₂ wie unter Formel I definiert ist, X das Anion einer anorganischen oder organischen Säure ist und n die Zahl 1 oder 2 bedeutet, in Gegenwart eines Lösungsmittels und einer starken Base.

Die in den Substituenten R₁ und R₂ vorkommenden Alkylgruppen können geradkettig oder verzweigt sein. Beispielsweise steht R₁ für Methyl, Ethyl, n-Propyl, iso-Propyl, n-Butyl, sek.-Butyl und tert.-Butyl, vorzugsweise jedoch für Methyl oder Ethyl. R₂ als C₁-C₈-Alkyl schließt die oben genannten Radikale ein und ferner die isomeren Pentyl-, Hexyl-, Heptyl-, und Octylradikale, wobei C₁-C₄-Alkylgruppen, insbesondere Methyl, Ethyl und iso-Propyl, bevorzugt sind.

C₁-C₄-Alkoxy schließt Methoxy, Ethoxy, n-Propoxy, iso-Propoxy und die vier isomeren Butoxy ein, steht aber insbesondere für Methoxy und Ethoxy.

Die gegebenenfalls substituierten Phenyl und Alkylphenylgruppen in den Bedeutungen von R₂ sind beispielsweise Phenyl, 2-Chlorphenyl, 3-Chlorphenyl, 4-Chlorphenyl, 2,4-Dichlorphenyl, 4-Fluorphenyl, 4-Methoxyphenyl, 2,4-Dimethoxyphenyl, 4-Trifluormethylphenyl, Benzyl, 4-Methylbenzyl, 4-Nitrobenzyl, 4-Brombenzyl, oder 2-Phenylethyl. Phenyl ist dabei bevorzugt.

Halogen als Substituent ist Fluor, Chlor oder Brom.

C₃-C₆-Cycloalkyl schließt Cyclopropyl, Cyclobutyl, Cyclopentyl und Cyclohexyl ein, wobei Cyclopropyl bevorzugt ist.

Ganz besonders bevorzugte Verbindungen der Formel I sind jene, in denen R₁ Methyl und R₂ Methyl, Ethyl oder Cyclopropyl bedeutet.

Bevorzugte Bedeutungen von X sind Halogenide, insbesondere Chloride, Sulfate, Bisulfate und Acetate.

Die N-cyanoimido-dialkylcarbonate der Formel II und Amidinsalze der Formel III sind bekannte Verbindungen oder können nach bekannten Verfahren hergestellt werden. Siehe beispielsweise The Chemistry of Amidines and Imidates, Saul Patai Ed., John Wiley & Sons, (1975).

Die Umsetzung eines N-cyanoimido-dialkylcarbonates mit einem Amidinsalzes zur Bildung eines 2-Amino-4-alkoxy-s-triazines wird in Gegenwart eines Lösungsmittels und einer starken Base durchgeführt.

Geeignete Lösungsmittel sind Alkohole der Formel R₁OH, in welcher R₁ vorzugsweise den R₁ Alkylgruppen des N-cyanoimido-dialkylcarbonates entspricht, offene und zyklische Ether wie Methyl-tert.-butylether oder Tetrafuran, C₃-C₇-Ketone wie Aceton und Methylisobutylketon, C₅-C₁₂-Kohlenwasserstoffe wie Hexan, C₁-C₆-Kohlenwasserstoffe, die durch 1-6 Halogenatome substituiert sind wie Methylenchlorid, Benzol oder durch 1 oder 2 Halogenatome oder C₁-C₃-Alkylgruppen substituiertes Benzol wie Toluol, oder Mischungen davon. Bevorzugte Mischungen enthalten einen Alkohol R₁OH mit mindestens einem der anderen Lösungsmittel. Wasser kann ebenfalls als eines der Lösungsmittel verwendet werden wenn das Amidin hydrolytisch stabil ist. Die Lösungsmittelmenge ist nicht kritisch solange die Menge ausreichend ist um gute Rührbarkeit sicherzustellen.

Geeignete starke Basen schließen die Alkalimetallalkoholate der Alkohole R₁OH ein. Alkalimetallhydroxide und Erdalkalimetallhydroxide können ebenfalls verwendet werden wenn das Amidin hydrolytisch stabil ist. Bevorzugt sind etwa stöchiometrische Mengen an Base.

Die Reaktionstemperaturen können von etwa -20°C bis +50°C, vorzugsweise von -10°C bis +25 °C variieren.

Die Reaktanden können in beliebiger Reihenfolge zugegeben werden. Bei einer bevorzugten Reakrionsvariante gibt man die Base unter Rühren und Kühlung zu einer Mischung des Amidinsalzes mit dem N-cyanoimido-dialkylcarbonat hinzu. Die Zugaberate ist nicht kritisch, sollte aber langsam genug sein um das oben erwähnte Temperaturintervall einzuhalten. Im Labor wurden gute Resultate mit einer Zugabezeit von 10 Minuten bis 2 Stunden erzielt. Wenn die Zugabe der Base beendet ist ist die Reaktion im wesentlichen vollständig.

Die Erfindung soll durch die folgenden, den Erfindungsgegenstand nicht beschränkenden Beispiele erläutert werden.

### Beispiel 1: 2-Amino-4-isopropyl-6-methoxy-s-triazin

A. In einen 100 ml Kolben gibt man 20 ml trockenes Methanol. Anschließend wird mit Eisbadkühlung mit trockenem HCl-Gas (etwa 10 g) gesättigt. Unter Rühren und Eisbadkühlung werden nun unter Beibehaltung einer Temperatur unter +10 °C 6,9 g (0,2 Mol) Isobutylnitril tropfenweise zugegeben. Man läßt die Lösung auf Raumtemperatur erwärmen und rührt das so hergestellte Methyl-isobutyrimidat für eine weitere Stunde.
B. In einen 250 ml Kolben gibt man 7,5 g (0.13 Mol) 30%ige wäßriges Ammoniak und 10 ml Wasser hinzu. Anschließend gibt man unter Eisbadkühlung und Beibehaltung einer Temperatur unter +10 °C und mit 25%ige NaOH eingestelltem pH von 9,5 bis 9,8 das Methyl-isobutyrimidat tropfenweise hinzu. Nach Beendigung de Imidatzugabe stellt man den pH auf 9,0 bis 9,5 ein und läßt eine Stunde bei Raumtemperatur rühren um schließlich Isobutyramidin-hydrochlorid zu erhalten.
C. Isobutyramidin-hydrochloridlösung wird im Eisbad gekühlt. Zu dieser Lösung gibt man 8 g (0,07 Mol) N-cyanoimido-dimethylcarbonat gefolgt von 5,8 g(0,073 Mol) tropfenweise zugegebener 50%iger NaOH. Anschließend läßt man die Mischung durch einstündiges Rühren Raumtemperatur erreichen. Nach Abdampfen des Methanols unter Vakuum schlämmt man die erhaltenen Feststoffe in 75 ml Wasser auf. Nach Kühlung, Filtration und anschließender Trocknung erhält man 7,2 g (61,2%) 2-Amino-4-isopropyl-6-methoxy-s-triazin mit einem Schmelzpunkt von +116 bis +117°C

### Beispiel 2: 2-Amino-4-methoxy-6-methyl-s-triazin

In einen 100 ml Kolben gibt man 5,5 g Acetamidin-hydrochlorid, 5,7 g N-cyanoimidodimethylcarbonat, 11 g Methanol und 11 g Toluol. Nach Kühlung der Reaktionsmischung auf +5 °C gibt man unter Beibehaltung einer Reaktionstemperatur von +5°C bis +10 °C 11,8 g 30%iges Natriummethylat tropfenweise hinzu. Nach beendeter Zugabe läßt man die Reaktionsmischung auf Raumtemperatur erwärmen. Anschließend filtert man ab und wäscht den Rückstand mit Methanol. Nach Aufschlämmen des Filterrückstandes in Wasser, Filtrieren, Waschen des Rückstandes mit Wasser und Trocknen erhält man 6,1 g (81 %) 2-Amino-4-methoxy-6-methyl-s-triazin mit einem Schmelzpunkt von + 256 bis +258°C.

### Beispiel 3: 2-Amino-4-ethoxy-6-methyl-s-triazin

IN einen 250 ml Rundkolben gibt man 5 g (0,05 Mol) 95%iges Acetamidin-hydrochlorid, 10 g (0,088 Mol) N-cyanoimido-diethylcarbonat und 50 g Ethanol. Nach Kühlung der gerührten Lösung auf 0 °C gibt man innerhalb von 30 Minuten eine Lösung von 4 g (0,056 Mol) Natriumethylat in 30 ml Ethanol hinzu. Anschließend läßt man die Reaktionsmischung auf Raumtemperatur erwärmen und rührt eine Stunde lang. Nach Abfiltrieren der Mischung dampft man vom Filtrat den Ethanol im Vakuum bis zum Erscheinen von Feststoffen ab. Der Filterrückstand wird mit diesen Feststoffen vereinigt und anschließend 50 ml Wasser zugegeben. Nach Rühren der Mischung filtriert man ab. Nach Trocknen des Rückstandes erhalt man 6,6 g (85,7%) 2-Amino-4-ethoxy-6-methyl-s-triazin mit einem Schmelzpunkt von +172 bis +174 °C.

### Beispiel 4: 2-Amino-4-cyclopropyl-6-ethoxy-s-triazin

In einen 250 ml Kolben gibt man bei einer Temperatur von + 5 °C 30 g einer wäßrigen Lösung von 0.05 Mol Cyclopropancarboxamidin-Hydrochlorid, 55 ml Methyl-tert.-butylether und 9 g N-cyanoimido-diethylcarbonat hinzu. Nach tropfenweiser Zugabe von 7 g 30%ige NaOH-Lösung läßt man die Reaktionsmischung auf Raumtemperatur erwärmen und rührt eine Stunde lang. Man gibt Wasser zu um das Salz zu lösen und die Phasen zu trennen. Nach zweimaliger Extraktion der wäßrigen Phase mit 20 ml Methyl-tert.-butylether, Vereinigung der Extrakte und Abdampfen des Lösungsmittels erhält man 6,8 g (75,5%) 2-Amino-4-cyclopropyl-6-ethoxy-s-triazin mit einem Schmelzpunkt von +103 bis +106 °C.

### Beispiel 5: 2-Amino-4-cyclopropyl-6-methoxy-s-triazin

In einen 250 ml Kolben gibt man 30 g einer wäßrigen Lösung von 0.05 Mol Cyclopropancarboxamidin-Hydrochlorid, 20 ml Methylenchlorid und 6,2 g (0,054 Mol) N-cyanoimido-dimethylcarbonat gelöst in 70 ml Methylenchlorid hinzu. Anschließend gibt man bei einer Temperatur von + 5 °C langsam 7 g (0,053 Mol) 30%ige NaOH-Lösung hinzu. Nach Entfernen der Kühlung wird eine Stunde lang gerührt. Nach Filtration und Trocknen des Rückstandes erhält man6,3 g (75,9%) 2-Amino-4-cyclopropyl-6-methoxy-s-triazin mit einem Schmelzpunkt von +162 bis +165 °C.

### Beispiel 6: 2-Amino-4-methoxy-6-phenyl-s-triazin

In einen 250 ml Kolben gibt man 9,2 g (0.05 Mol) 85%iges Benzamidin-hydrochlorid, 75 ml Methanol und 6 g (0,053 Mol) N-cyanoimido-dimethylcarbonat. Anschließend gibt man bei einer Temperatur von + 5 °C tropfenweise 7 g (0,053 Mol) 30%ige NaOH-Lösung hinzu. Nach einstündigem Rühren bei Raumtemperatur dampft man das Methanol ab und schlämmt die Feststoffe in 75 ml Wasser auf. Nach Filtration und Trocknung erhält man 9,2 g (91 %) 2-Amino-4-methoxy-6-phenyl-s-triazin mit einem Schmelzpunkt von +159 bis +160 °C.

## Patentansprüche

1. Verfahren zur Herstellung von 2-Amino-4-alkoxy-s-triazinen der Formel I 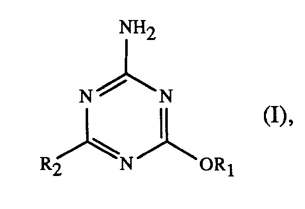 worin
R₁ für C₁-C₄-Alkyl oder C₁-C₂-Alkoxy-C₁-C₂-alkyl; und
R₂ für C₁-C₈-Alkyl, welches unsubstituiert ist oder durch C₁-C₄-Alkoxy, Phenyl oder durch C₁-C₄-Alkyl, C₁-C₄-Alkoxy, Difluormethyl, Trifluormethyl, Halogen oder Nitro substituiertes Phenyl substituiert ist; Phenyl oder durch C₁-C₄-Alkyl, C₁-C₄-Alkoxy, Difluormethyl, Trifluormethyl, Halogen oder Nitro substituiertes Phenyl; C₃-C₆-Cycloalkyl oder Allyl stehen, dadurch gekennzeichnet, daß man ein N-cyanoimido-dialkylcarbonat der Formel II 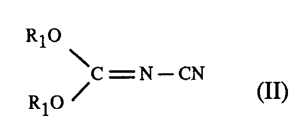 worin R₁ wie in Formel I definiert ist; mit einem Amidinsalz der Formel III 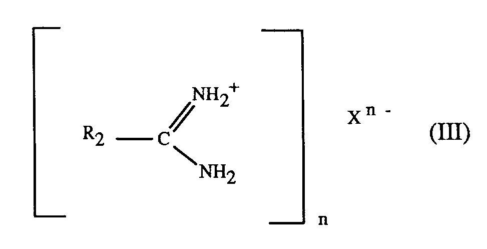 in welcher R₂ wie unter Formel I definiert ist, X das Anion einer anorganischen oder organischen Säure ist und n die Zahl 1 oder 2 bedeutet, in Gegenwart eines Lösungsmittels und einer Starken Base umsetzt.

2. Verfahren nach Anspruch 1, dadurch gekennzeichnet, daß R₁ Methyl oder Ethyl und R₂ C₁-C₄-Alkyl, Cyclopropyl oder Phenyl bedeutet.

3. Verfahren nach Anspruch 2, dadurch gekennzeichnet, daß R₁ Methyl und R₂ Methyl, Ethyl oder Cyclopropyl bedeuten.

4. Verfahren nach Anspruch 3, dadurch gekennzeichnet, daß R₁ und R₂ Methyl bedeuten.

5. Verfahren nach Anspruch 1, dadurch gekennzeichnet, daß n die Zahl 1 bedeutet und X für ein Halogenid-, Bisulfat- oder Acetatanion steht oder wenn n die Zahl 2 bedeutet X für das Sulfatanion steht.

6. Verfahren nach Anspruch 5, dadurch gekennzeichnet, daß n die Zahl 1 bedeutet und X für das Chloridanion steht.

7. Verfahren nach Anspruch 1, dadurch gekennzeichnet, daß man als Lösungsmittel Wasser, ein Alkohol der Formel R₁OH, worin R₁ wie unter Formel I definiert ist, ein offenkettiger oder zyklischer Ether, ein C₃-C₇-Keton, ein C₅-C₁₂-Kohlenwasserstoff, ein C₁-C₆-Kohlenwasserstoff, der durch 1-6 Halogenatome substituiert ist, Benzol oder durch 1 oder 2 Halogenatome oder C₁-C₃-Alkylgruppen substituiertes Phenyl, oder Mischungen davon verwendet.

8. Verfahren nach Anspruch 7, dadurch gekennzeichnet, daß man als Lösungsmittel Wasser, Methanol, Ethanol, Methyl-tert.-butylether, Toluol oder Methylenchlorid oder eine Mischung davon verwendet.

9. Verfahren nach Anspruch 8, dadurch gekennzeichnet, daß man als Lösungsmittel eine Mischung aus Methanol und Toluol verwendet.

10. Verfahren nach Anspruch 1, dadurch gekennzeichnet, daß man als starke Base das Alkalimetallsalz eines C₁-C₄-Alkohols verwendet.

11. Verfahren nach Anspruch 1, dadurch gekennzeichnet, daß man als starke Base das Alkalimetallhydroxid oder Erdalkalimetallhydroxid verwendet.

12. Verfahren nach Anspruch 11, dadurch gekennzeichnet, daß man als starke Base festes Natriumhydroxid oder in Wasser gelöstes Natriumhydroxid verwendet.

13. Verfahren nach Anspruch 1, dadurch gekennzeichnet, daß man es bei einer Temperatur von -20°C bis +50°C durchführt.

14. Verfahren nach Anspruch 1, dadurch gekennzeichnet, daß man es bei einer Temperatur von -10°C bis +25°C durchführt.

15. Verfahren nach Anspruch 1, dadurch gekennzeichnet, daß man eine methanolische Lösung von Natrimmethylat zu einer gerührten Mischung von N-cyanoimido-dimethylcarbonat und Acetamidin-Hydrochlorid in Methanol und Toluol als Lösungsmittel bei einer Temperatur im Bereich von +5 bis +10 °C zugibt und rührt bis die Reaktion im wesentlichen vollständig ist.

## Claims

1. A process for the preparation of a 2-amino-4-alkoxy-s-triazine of formula I 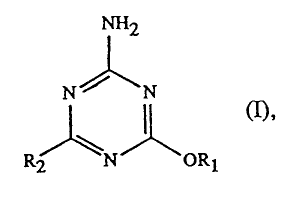 wherein
R₁ is C₁-C₄alkyl or C₁-C₂alkoxy-C₁C₂alkyl; and
R₂ is C₁-C₈alkyl which is unsubstituted or substituted by C₁-C₄alkoxy, phenyl or by phenyl substituted by C₁-C₄alkyl, C₁-C₄alkoxy, difluoromethyl, trifluoromethyl, halo or by nitro; phenyl or phenyl substituted by C₁-C₄alkyl, C₁-C₄alkoxy, difluoromethyl, trifluoromethyl, halo or by nitro; C₃-C₆cycloalkyl or allyl,
which process comprises reacting, in the presence of a solvent and a strong base, a dialkyl (N-cyanoimido)carbonate of the formula II 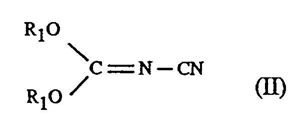 wherein R₁ is as defined for formula I, with an amidine salt of the formula III 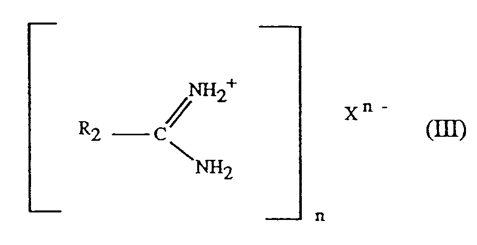 wherein R₂ is as defined for formula I, X is the anion of an inorganic or organic acid and n is the number 1 or 2.

2. A process according to claim 1, wherein R₁ is methyl or ethyl and R₂ is C₁-C₄alkyl, cyclopropyl or phenyl.

3. A process according to claim 2, wherein R₁ is methyl and R₂ is methyl, ethyl or cyclopropyl.

4. A process according to claim 3, wherein R₁ and R₂ are each methyl.

5. A process according to claim 1, wherein n is the number 1 and X is a halide, bisulfate or acetate anion or, when n is the number 2, X is the sulfate anion.

6. A process according to claim 5, wherein n is the number 1 and X is the chloride anion.

7. A process according to claim 1, wherein the solvent is water, an alcohol of the formula R₁OH wherein R₁ is as defined for formula I, an open-chain or cyclic ether, a C₃-C₇ketone, a C₅-C₁₂hydrocarbon, a C₁-C₆hydrocarbon substituted by 1-6 halogen atoms, benzene, or phenyl substituted by 1 or 2 halogen atoms or C₁-C₃alkyl groups, or a mixture thereof.

8. A process according to claim 7, wherein the solvent is water, methanol, ethanol, methyl tert-butyl ether, toluene or methylene chloride, or a mixture thereof.

9. A process according to claim 8, wherein the solvent is a mixture of methanol and toluene.

10. A process according to claim 1, wherein the strong base is an alkali metal salt of a C₁-C₄alcohol.

11. A process according to claim 1, wherein the strong base is an alkali metal or alkaline earth metal hydroxide.

12. A process according to claim 11, wherein the strong base is solid sodium hydroxide or sodium hydroxide dissolved in water.

13. A process according to claim 1, wherein the temperature is in the range of -20°C to +50°C.

14. A process according to claim 1, wherein the temperature is in the range of -10°C to +25°C.

15. A process according to claim 1, which comprises adding a methanolic solution of sodium methylate to a stirred mixture of dimethyl (N-cyanoimido)carbonate and acetamidine hydrochloride, in methanol and toluene as solvent, at a temperature in the range of +5 to +10°C, and stirring until the reaction is essentially complete.

## Revendications

1. Procédé pour la préparation de 2-amino-4-alcoxy-triazines-s de formule I 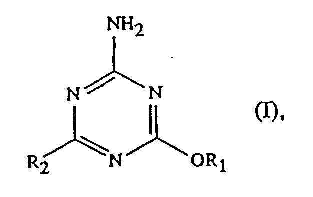 dans laquelle
R₁ représente un groupe alkyle en C₁-C₄ ou alcoxy(C₁-C₂)-alkyle(C₁-C₂) et
R₂ représente un groupe alkyle en C₁-C₈ qui n'est pas substitué ou qui est substitué par un radical alcoxy en C₁-C₄, phényle ou par un radical phényle substitué par un atome d'halogène ou par un groupe alkyle en C₁-C₄, alcoxy en C₁-C₄, difluorométhyle, trifluorométhyle ou nitro; un groupe phényle ou un groupe phényle substitué par un atome d'halogène ou par un groupe alkyle en C₁-C₄, alcoxy en C₁-C₄, difluorométhyle, trifluorométhyle ou nitro; un groupe cycloalkyle en C₃-C₆ ou allyle,
caractérisé en ce que l'on fait réagir un N-cyano-imido-carbonate de dialkyle de formule II 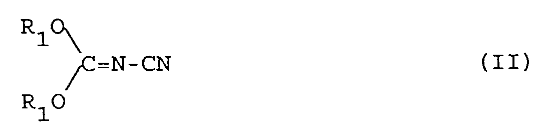 dans laquelle R₁ est tel que défini dans la formule I, avec un sel d'amidine de formule III 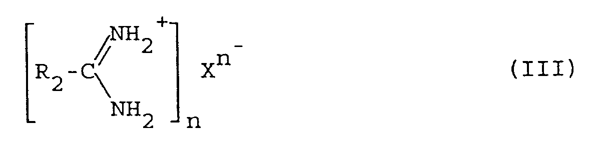 dans laquelle R₂ est tel que défini dans la formule I, X est l'anion d'un acide organique ou minéral, et n représente le nombre 1 ou 2, en présence d'un solvant et d'une base forte.

2. Procédé selon la revendication 1, caractérisé en ce que R₁ représente le groupe méthyle ou éthyle et R₂ représente un groupe alkyle en C₁-C₄, cyclopropyle ou phényle.

3. Procédé selon la revendication 2, caractérisé en ce que R₁ représente le groupe méthyle et R₂ représente le groupe méthyle, éthyle ou cyclopropyle.

4. Procédé selon la revendication 3, caractérisé en ce que R₁ et R₂ représentent des groupes méthyle.

5. Procédé selon la revendication 1, caractérisé en ce que n représente le nombre 1 et X représente un anion halogénure, bisulfate ou acétate, ou, lorsque n représente le nombre 2, X représente l'anion sulfate.

6. Procédé selon la revendication 5, caractérisé en ce que n représente le nombre 1 et X représente l'anion chlorure.

7. Procédé selon la revendication 1, caractérisé en ce que l'on utilise comme solvant l'eau, un alcool de formule R₁OH dans laquelle R₁ est tel que défini sous la formule I, un éther cyclique ou à chaîne droite, une cétone en C₃-C₇, un hydrocarbure en C₅-C₁₂, un hydrocarbure en C₁-C₆ qui est substitué par 1-6 atomes d'halogène, le reste benzénique ou un radical phényle substitué par 1 ou 2 atomes d'halogène ou groupes alkyle en C₁-C₃, ou des mélanges de ceux-ci.

8. Procédé selon la revendication 7, caractérisé en ce que l'on utilise comme solvant l'eau, le méthanol, l'éthanol, l'éther méthyl-tert-butylique, le toluène ou le chlorure de méthylène, ou un mélange de ceux-ci.

9. Procédé selon la revendication 8, caractérisé en ce que l'on utilise comme solvant un mélange de méthanol et de toluène.

10. Procédé selon la revendication 1, caractérisé en ce que l'on utilise comme base forte un sel alcalin d'un alcool en C₁-C₄.

11. Procédé selon la revendication 1, caractérisé en ce que l'on utilise comme base forte un hydroxyde de métal alcalin ou un hydroxyde de métal alcalino-terreux.

12. Procédé selon la revendication 11, caractérisé en ce que l'on utilise comme base forte l'hydroxyde de sodium solide ou l'hydroxyde de sodium dissous dans l'eau.

13. Procédé selon la revendication 1, caractérisé en ce qu'on l'effectue à une température de -20 à +50°C.

14. Procédé selon la revendication 1, caractérisé en ce qu'on l'effectue à une température de -10 à +25°C.

15. Procédé selon la revendication 1, caractérisé en ce que l'on ajoute une solution méthanolique de méthylate de sodium à un mélange agité de N-cyano-imidocarbonate de diméthyle et de chlorhydrate d'acétamidine dans du méthanol et du toluène en tant que solvant, à une température dans la plage de +5 à +10°C, et on l'agite jusqu'à ce que la réaction soit pratiquement totale.
